# EUROPÄISCHE PATENTSCHRIFT

(11) **EP 2 818 127 B1**
(45) Veröffentlichungstag und Bekanntmachung des Hinweises auf die Patenterteilung: **16.03.2016**
(21) Anmeldenummer: 14155440.2
(22) Anmeldetag: 20.06.2013
(51) Int. Cl.: A61B 18/14, A61B 5/00

(54) **Elektrochirurgisches Instrument mit Lichtleiter**
Electrosurgical instrument with light guide
Instrument électrochirurgical doté d'un guide de lumière

(43) Veröffentlichungstag der Anmeldung: 31.12.2014
(62) Teilanmeldung aus: 13173066.5
(73) Patentinhaber: ERBE Elektromedizin GmbH, 72072 Tübingen (DE)
(72) Erfinder: Spether, Dominik, 72074 Tübingen (DE); Fischer, Klaus, 72202 Nagold (DE); Neugebauer, Dr. Alexander, 72116 Mössingen (DE); Enderle, Markus, 72070 Tübingen (DE)
(74) Vertreter: Rüger, Barthelt & Abel

(56) Entgegenhaltungen:
- WO-A2-2011/055369
- US-A- 5 769 791
- US-A1- 2007 213 704
- US-A1- 2012 296 205
- US-A1- 2012 296 238
- US-A1- 2012 316 558

## Beschreibung

Die Erfindung betrifft ein Instrument zur elektrochirurgischen Einwirkung auf biologisches Gewebe und insbesondere ein Instrument zur HF-Chirurgie.

Es sind elektrochirurgische Instrumente bekannt, die unter Funkenerzeugung auf biologisches Gewebe einwirken und das dabei entstehende Licht einer Analyseeinrichtung zuführen.

Dazu offenbart die WO 2011/055369 A2 einen Mikroplasmakopf für medizinische Anwendungen. Dieser Mikroplasmakopf wird durch ein längliches flexibles Instrument gebildet, an dessen Ende Elektroden zur Plasmaerzeugung vorgesehen sind. Etwas zurückgesetzt endet ein Lichtleiter, dessen offene Stirnfläche ein Lichteintrittsfenster bildet. Vor dem Lichteintrittsfenster bildet sich ein Plasma, dessen Licht von dem Lichtleiter aufgenommen wird. Die angeschlossene Lichtanalyseeinrichtung untersucht das Lichtspektrum des von dem Plasma ausgehenden Lichts insbesondere auf Vorhandensein einer charakteristischen Phosphorlinie. Dies dient der Unterscheidung lebenden Gewebes von Plaques bei der Anwendung des Instruments zur Beseitigung von Plaques in Blutgefäßen.

Eine subtilere Lichtuntersuchung wird von der US 2007/0213704 A1 vorgeschlagen. Das dort veranschaulichte längliche Instrument weist mittig eine Lichtleitfaser auf, die in einer sphärischen Ausnehmung eines keramischen Endstücks endet. In dieser Ausnehmung sind außerdem zwei scharfkantige Elektroden angeordnet, die in dieser Ausnehmung ein Plasma erzeugen.

Eine erfolgreiche spektrale Lichtanalyse setzt voraus, dass das Licht zu dem Spektralanalysator gelangt.

Es ist Aufgabe der Erfindung, dies sicherzustellen.

Diese Aufgabe wird mit dem Instrument nach Anspruch 1 gelöst:
Das erfindungsgemäße Instrument weist mindestens eine Elektrode auf, die über eine Leitung mit einer elektrischen Quelle verbindbar oder verbunden ist. Die Quelle kann z.B. ein HF-Generator sein. Die Elektrode kann monopolar sein und aus einem oder mehreren Teilen bestehen. In diesem Fall wird die zum Schließen eines elektrischen Stromkreises nötige Gegenelektrode als Neutralelektrode an dem Patienten befestigt.

Das Instrument kann ein für den offenchirurgischen Einsatz vorgesehenes Instrument oder als für den laparoskopischen Einsatz vorgesehenes Instrument ausgebildet sein. Es ist insbesondere für HF-chirurgische Eingriffe hergerichtet, bei denen an der Elektrode Funken, vorteilhaft mit einem Spektrum zwischen 200nm und 1200nm, entstehen. Als elektrische Quelle ist in solchen Fällen vorzugsweise ein HF-Generator vorgesehen.

Das erfindungsgemäße Instrument ist zum Anschluss an eine Lichtanalyseeinrichtung vorgesehen, mit der aus Merkmalen des aufgenommenen Lichts Informationen über das von dem Funken erfasste biologische Gewebe gewonnen werden. Zur Aufnahme dieses Lichts ist ein Lichteintrittsfenster vorgesehen, das durch einen Fluidkörper gebildet ist. Dieser steht in Berührung mit dem Lichtleiter, um das aufgenommene Licht in den Lichtleiter und über diesen an die Lichtanalyseeinrichtung zu übertragen. Der Fluidkörper hat eine flüssige Oberfläche, die auch dann, wenn sie sich in unmittelbarer Nähe zu dem Funken befindet nicht durch Niederschlag von Rauchteilchen verschmutzt oder verschmort. Es ist deswegen möglich, das von dem Funken ausgehende Licht ohne spektrale Verfälschung aufzunehmen.

Insbesondere wenn das Lichteintrittsfenster in unmittelbarer Nachbarschaft zu der Elektrode angeordnet ist, kann sichergestellt werden, dass das von dem Funken ausgehende Licht wirklich aufgenommen und nicht durch zwischen dem Funken und dem Lichteintrittsfenster liegende Gewebeteile, Rauch oder Ähnliches verdeckt wird. Im bevorzugten Fall kann das Lichteintrittsfenster die Elektrode berührend angeordnet sein, beispielsweise indem ein Fluidkörper durch Adhäsion zwischen der Elektrode und dem Lichtleiter gehalten ist. Dazu ist es vorteilhaft, wenn das Lichteintrittsfenster und/oder die Elektrode eine hydrophile Oberfläche aufweisen. Auf diese Weise kann ein im Wesentlichen ruhender Fluidkörper zwischen einer Fläche des Lichtleiters und der Elektrode gehalten sein. Im bevorzugten Fall bilden dazu zwei einander gegenüberliegende hydrophile Elektrodenflächen, zwischen denen keine Potentialdifferenz besteht, einen Spalt, dessen Boden von einer hydrophilen Lichtleiterfläche gebildet ist. Die Spaltweite ist vorzugsweise so gering, dass der Fluidkörper in dem Spalt durch Kapillarwirkung gehalten wird. Das den Fluidkörper bildende Fluid kann in der Umgebung vorhandenes Spülfluid, Gewebeflüssigkeit oder auch ein über einen Kanal hereingeführtes Fluid sein. Der Kanal kann sich zum Beispiel durch den Lichtleiter erstrecken.

Das Lichteintrittsfenster kann auch durch einen strömenden Fluidkörper gebildet sein, beispielsweise indem aus der Elektrode ein transparenter Fluidstrahl austritt. Zum Beispiel kann der Lichtleiter in einem Fluidkanal angeordnet sein, der Fluid an die Fluidaustrittsöffnungen heranführt, aus denen dann Fluidstrahlen austreten. Diese bilden die Lichteintrittsfenster und leiten von dem Funken herrührendes Licht in den Fluidkanal, wo es weiter von dem Lichtleiter aufgenommen wird.

Unabhängig davon, ob der das Lichteintrittsfenster bildende Fluidkörper ruhend oder strömend vorgesehen ist, ist es zweckmäßig, wenn die Elektrode mindestens zwei elektrische miteinander verbundene Bereiche aufweist, zwischen denen das Lichteintrittsfenster angeordnet ist. Der Fluidkörper kann somit zwischen den auf gleichem Potential liegenden Elektrodenflächen in unmittelbarster Nähe zu dem entstehenden Funken angeordnet sein.

Bei einer besonders bevorzugten Ausführungsform weist das Instrument einen Lichtleiter mit einem starren Abschnitt auf, der die Elektrode trägt. Der starre Abschnitt kann zum Beispiel in eine sich verjüngende Spitze übergehen, an der eine aus einem oder mehreren Drähten, Metallstreifen oder dergleichen bestehende Elektrode angeordnet ist. Der Winkel der sich verjüngende Spitze kann, beispielsweise 5 bis 90 Grad betragen. Die Größe des Winkels wird kann abhängig vom Brechungsindex des verwendeten Lichtleiters z.B. bei Verwendung von Glas mit 1,46 und abhängig vom Brechungsindex des umgebenen Mediums des Lichtleiters wie Luft (1,0) oder Wasser (1,33) bestimmt.

Vorzugsweise ist die Elektrode durch einen Draht gebildet, der die sich verjüngende Spitze in mehreren Windungen umfasst. In den Abständen zwischen einander benachbarten Windungen kann sich ein Fluidkörper durch Adhäsion und gegebenenfalls Kapillarwirkung halten. Er bildet das Lichteinrittsfenster und überträgt Licht auf den Lichtleiter. Unabhängig davon, an welcher Stelle der kegelförmigen Drahtwindungen ein Funke entspringt, wird das entstehende Licht von dem Lichtleiter zu einem großen Teil aufgenommen und kann somit unverfälscht der Analyseeinrichtung zugeführt werden. Der Lichtleiter kann beispielsweise mittels eines lichtleitenden Materials wie z.B. Glas, Kunststoff ausgebildet sein. Der Lichtleiter kann auch aus einem geeigneten innen verspiegelten Rohr oder Kanüle bestehen, welches alternativ mit stehendem oder strömendem Fluid gefüllt sein kann. Weitere Einzelheiten vorteilhafter Ausführungsformen der Erfindung sind Gegenstand der Zeichnung, der Beschreibung oder von Ansprüchen. Es zeigen:
Figur 1 eine elektrochirurgische Einrichtung mit einem erfindungsgemäßen Instrument, in schematisierter Darstellung,
Figur 2 eine lichtleitende Elektrode des Instruments nach Figur 1, in vereinfachter Perspektivdarstellung,
Figur 3 ein Ende eines Lichtleiters zum Aufbau einer lichtleitenden Elektrode, in Seitenansicht,
Figur 4 das Ende der Elektrode nach Figur 2, in Seitenansicht,
Figur 5 eine ausschnittsweise Darstellung der Spitze der Elektrode nach Figur 4, in vergrößerter Schnittdarstellung,
Figur 6 eine abgewandelte Ausführungsform einer licht leitenden Elektrode mit Fluidkanal,
Figur 7 den lichtleitenden Träger für die Elektrode nach Figur 6, in schematisierter Seitenansicht,
Figur 8 eine Ausführungsform für eine Elektrode mit ausströmenden Fluidstrahlen als Lichteintrittsfenster, in teilweiser aufgeschnittener Perspektivdarstellung,
Figur 8b eine Ausführungsform einer, mit einer isolierendem Werkstoff beschichteten Elektrode gemäß Figur 8, in geschnittener Darstellung,
Figur 9 eine weiter abgewandelte Ausführungsform einer erfindungsgemäßen Elektrode mit Lichtleiter, in Perspektivdarstellung, und
Figur 10 die Elektrode nach Figur 9, im Querschnitt.

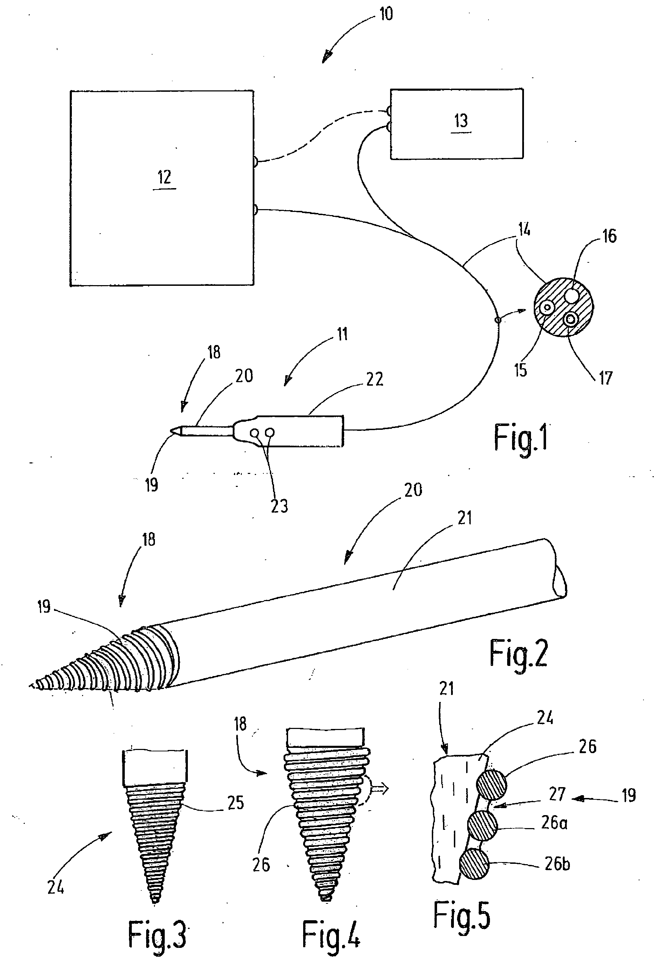

In Figur 1 ist eine elektrochirurgische Einrichtung 10 veranschaulicht, zu der ein vom Anwender zu führendes Instrument 11, ein speisendes Gerät 12 und eine Lichtanalyseeinrichtung 13 gehören. Diese kann, wie dargestellt, als separater Einheit oder auch als Bestandteil des Geräts 12 ausgebildet sein. Zur Verbindung des Instruments 11 mit dem Gerät 12 und der Lichtanalyseeinrichtung 13 sind entsprechende Leitungen 14 vorgesehen, die mindestens eine elektrische Leitung 15, einen Lichtleiter 16 und optional mindestens eine Fluidleitung 17 enthält. Die elektrische Leitung 15 verbindet eine Elektrode 18 des Instruments 11 mit dem speisenden Gerät 12, das beispielsweise einen HF-Generator enthält. Der Lichtleiter 16 verbindet ein an der Elektrode 18 vorgesehenes Lichteintrittsfenster 19 mit der Lichtanalyseeinrichtung 13.

Das Instrument 11 kann als laparoskopisches Instrument oder, wie es in Figur 1 symbolisch veranschaulicht ist, auch als Instrument für offenchirurgische Eingriffe ausgebildet sein. Es weist einen Elektrodenträger 20 auf, der in Figur 2 gesondert veranschaulicht ist. Dieser wird beispielsweise durch einen starren Lichtleiter 21 gebildet, der an den Lichtleiter 16 der Leitung 14 angeschlossen ist. Der Anschluss kann innerhalb eines Griffs 22, der auch ein oder mehrere Bedienelemente 23 tragen kann, oder von diesem Griff beabstandet verwirklicht sein. Der von dem starren Lichtleiter 21 gebildete Elektrodenträger kann, wie es in Figur 3 veranschaulicht ist, an einem Ende 24 kegelförmig verjüngt ausgebildet sein. An diesem Ende 24 kann eine ein- oder mehrgängige Wendelnut 25 ausgebildet sein, deren Ganghöhe größer ist als die Breite eines von der Wendelnut 25 aufzunehmenden Drahts. Die Figur 4 veranschaulicht einen die Elektrode 18 bildenden Draht 26, der schraubenförmig gewickelt in die Wendelnut 25 eingelegt ist. Dieser Draht 26 bildet die eigentliche Elektrode. Er ist über eine oder mehrere sich entlang des starren Lichtleiters 21 erstreckende Drähte mit der Leitung 15 verbunden.

Die Verhältnisse sind in Figur 5 nochmals gesondert veranschaulicht. Zwischen benachbarten Windungen 26a, 26b des Drahts 26 besteht ein Abstand, so dass dort der Lichtleiter 21 bzw. sein Ende 24 sichtbar bleibt. Die Oberfläche des Lichtleiters ist vorzugsweise hydrophil ausgebildet. Außerdem ist vorzugsweise die Oberfläche des Drahts 26 wenigstens in dem an den Lichtleiter 21 angrenzenden Teil hydrophil ausgebildet. Dies bedeutet, dass der zwischen den Windungen des Drahts 26 freiliegende Teil des Lichtleiters 21 und die angrenzenden Teile der Drähte 26 von Wasser leicht benetzt werden und einen Wasserkörper durch Adhäsion festhalten.

In Figur 5 ist veranschaulicht, dass zwischen benachbarten Windungen des Drahts 26 ein Fluidkörper 27 durch Adhäsion gehalten ist. Ist der Spalt zwischen den Windungen 26a, 26b eng genug, kann auch von Kapillarwirkung gesprochen werden. Außerdem verbreitert sich der Spalt wegen des kreisförmigen Querschnitts des Drahtes 26 zu dem Lichtleiter hin.

Die hydrophile Eigenschaft der vom Fluidkörper 27 berührten Oberflächen ist durch die konkave Wölbung seiner nach außen gerichteten Oberfläche erkennbar. Der Fluidkörper 27 besteht im Wesentlichen aus Wasser, beispielsweise Spülflüssigkeit, Gewebsflüssigkeit oder dergleichen. Er bildet das Lichteintrittsfenster 19, über das Licht eines von dem Draht 26 ausgehenden Funkens aufgenommen und an den Lichtleiter 21 übergeben wird.

Im vorliegenden Fall handelt es sich um einen ruhenden Fluidkörper 27, der durch aus der Umgebung zufließende Flüssigkeit gebildet ist. Auf seiner flüssigen Oberfläche können sich Rauch oder sonstige Feststoffpartikel nicht dauerhaft halten. In Betrieb ist somit sichergestellt, dass das von einem Funken ausgehende Licht unverfälscht zu der Lichtanalyseeinrichtung 13 gelangt. Unter "Licht" wird dabei nicht nur sichtbares, sondern, falls gewünscht, auch infrarotes Licht und/oder ultraviolettes Licht verstanden.

Die insoweit beschriebene elektrochirurgische Einrichtung 10 arbeitet wie folgt:
Der Anwender nutzt das Instrument 11, um elektrochirurgische, vorzugsweise HF-elektrochirurgische Eingriffe vorzunehmen. Eine nicht weiter veranschaulichte Neutralelektrode, die mit dem Gerät 12 verbunden ist, ist am Patienten befestigt. Der Anwender kann nun mit der Elektrode 18 einen Funken zu dem Gewebe eines Patienten erzeugen und dadurch einen Effekt, beispielsweise Schneiden, Koagulieren oder dergleichen bewirken. Das von dem Funken erzeugte Licht wird auf subtile Weise von dem behandelten Gewebe beeinflusst. Teile des von dem Funken bearbeiteten Gewebes, Moleküle, Molekülbruchstücke, Atome, Ionen gelangen in den Funken und erzeugen eine Lichtemmission. Dieses Licht wird über das Lichteintrittsfenster 19 aufgenommen und über den Lichtleiter 21, 16 an die Lichtanalyseeinrichtung 13 geliefert. Diese nimmt eine Spektralzerlegung des Lichts vor und analysiert das Spektrum des aufgenommenen Lichts, um möglichst präzise die Art des behandelten Gewebes zu ermitteln. Entsprechende vom Behandler sichtbare, hörbare oder fühlbare Signale können dann den Gewebetyp oder einen Wechsel des Gewebetyps anzeigen.

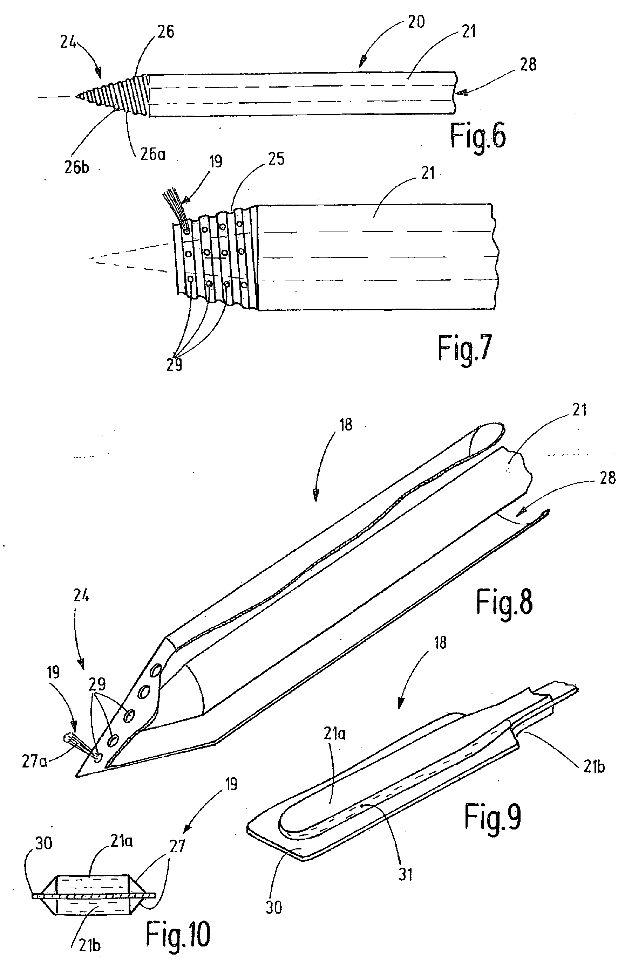

Es wird darauf hingewiesen, dass zahlreiche Abwandlungen möglich sind. Beispielsweise kann die Lichtanalyseeinrichtung 13 Bestandteil des Instruments 11 sein. Sie kann zum Beispiel in dessen Griff 22 eingebaut sein. Weitere Abwandlungen können die Elektrode 18, den Elektrodenträger 20 und den Fluidkörper 27 betreffen. Dazu veranschaulicht Figur 6 einen Elektrodenträger 20. Dieser ist vorzugsweise aus einem lichtleitenden Material ausgebildet. Er bildet somit den starren Lichtleiter 21. In dem starren Lichtleiter 21 ist hier ein Fluidkanal 28 vorgesehen, durch das ein geeignetes Fluid, beispielsweise physiologische Kochsalzlösung, zu dem Ende 24 geführt wird. Es können dort eine oder vorzugsweise mehrere Fluidaustrittsöffnungen 29 vorgesehen sein, die mit dem Fluidkanal 28 verbunden sind. Die Fluidaustrittsöffnungen 29 sind vorzugsweise so angeordnet, dass sie zwischen den voneinander beabstandeten Windungen 26a, 26b des Drahts 26 münden, d.h. dass sie zwischen der Wendelnut 25 liegen, wie es Figur 7 zeigt.

Bei einem solchen Instrument kann während des Betriebs der Fluidkörper 27 über die Fluidaustrittsöffnungen 29 fortwährend erneuert werden. Es kann in Abhängigkeit vom Fluiddruck auch bewirkt werden, dass aus den Fluidaustrittsöffnungen 29 Fluidstrahlen austreten, die dann als Lichteintrittsfenster 19 wirken. Diese stehen in unmittelbarer Verbindung zu den an dem Draht 26 entspringenden Funken und nehmen somit dessen Licht auf. Bei dieser Ausführungsform ist es auch möglich, anstelle des starren Lichtleiters 21 optisch undurchsichtiges Material zu verwenden und den Lichtleiter in dem Fluidkanal 28 anzuordnen, um dort Licht aus dem Fluid aufzunehmen.

Es wird darauf hingewiesen, dass bei allen vorstehend beschriebenen Ausführungsformen nach Figur 1 bis 7 der starre Lichtleiter 21 auch als flexibler Lichtleiter ausgebildet sein kann. Dies trifft insbesondere für laparoskopisch einzusetzende Instrumente 11 zu.

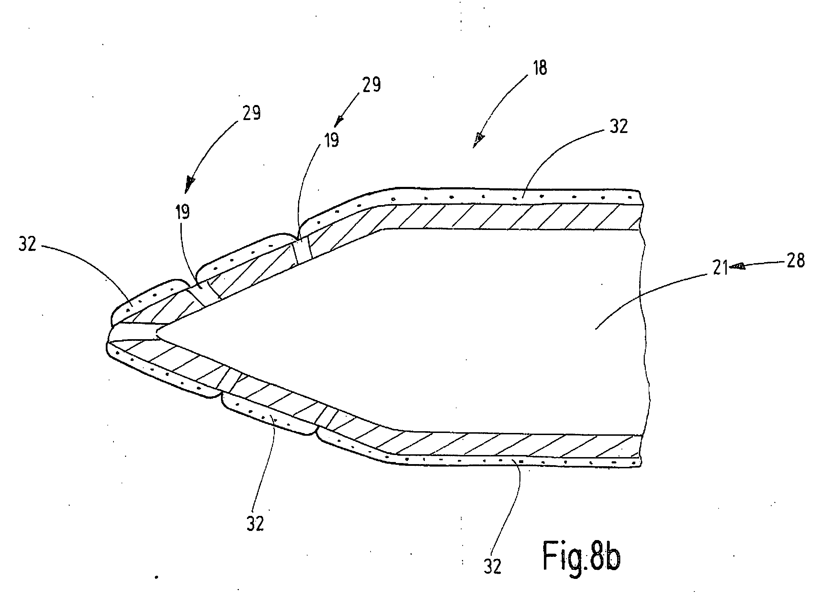

Eine weitere Ausführungsform des erfindungsgemäßen Instruments 11 geht aus Figur 8 hervor. Die Elektrode 18 ist als dünnwandiges Röhrchen elektrisch leitend oder durch partiell aufgebrachte elektrisch leitende Metallschichten ausgebildet, an dessen Ende 24 Fluidaustrittsöffnungen 29 ausgebildet sind. Über den Fluidkanal 28 wird Fluid zum Beispiel physiologische Kochsalzlösung oder ein anderes auf Wasser basierendes Fluid zu den Fluidaustrittsöffnungen 29 geleitet. Die dort austretenden Fluidstrahlen 27a bilden die Lichteintrittsfenster 19. Das Fluid im Fluidkanal 28 kann den Lichtleiter 21 bilden. Ist der Fluiddruck geringer, so dass das Fluid aus den Fluidaustrittsöffnungen 29 nur austropft oder aussickert, bilden die flüssigkeitsgefüllten Fluidaustaustrittsöffnungen 29 die Lichteintrittsfenster 19.

In dem Kanal 28 ist der Lichtleiter 21 angeordnet, der sich vorzugsweise bis zu dem Ende 24 erstreckt. Er wirkt als Lichtsammler um das über die Fluidaustrittsöffnungen 29 in den Kanal 28 gelangte Licht zu erfassen und der Lichtanalyseeinrichtung 13 zuzuführen.

Das Ende 24 der Elektrode 18, wie auch das Ende des Lichtleiters 21, können, wie dargestellt, kegelförmig oder bedarfsweise auch anderweitig ausgeformt sein, beispielsweise kugelförmig, spatelförmig oder dergleichen.

Die Lichtemissionserfassung kann verbessert werden, wenn der Funke im Bereich der Lichteintrittsfenster 19 entsteht. Dazu kann das Ausführungsbeispiel gemäß Figur 8 eine elektrisch isolierende Schicht wie in Figur 8b dargestellt aufweisen. Das Aufbringen dieser elektrischen isolierenden Schicht, beispielsweise einer Kunststoffschicht, auf die Elektrode 18 mit Öffnungen, die die Lichteintrittsfenster 19 bilden, führt zu unterschiedlichen Dicken der Isolationsschicht. Im Kantenbereich der Öffnungen der Lichteintrittsfenster entsteht eine Isolationsschicht mit geringerer Dicke. Diese mit dünner Schicht überzogenen Stellen werden beim Anlegen einer HF-Spannung im Bereich von 1000 bis 10000 Volt bevorzugt durchschlagen, wodurch der Funke zwischen dem Gewebe und der Elektrode unmittelbar an den Lichteintrittsfenster 19 entsteht. Dadurch gelingt es, den Entstehungsort des Funkens und somit der Lichtemission unmittelbar an der Kante im Bereich des Lichteintrittsfensters 19 festzulegen. Der Weg zum Lichtleiter 29 ist so gering wie nur möglich. Die Lichtemissionserfassung kann dadurch nahezu ohne Verluste erfolgen.

Eine weiter abgewandelte Ausführungsform zeigt Figur 9. Die Elektrode 18 weist ein elektrisch leitendes ebenes Plättchen 30 auf, das mit der elektrischen Quelle verbunden ist. Auf mindestens einer, vorzugsweise beiden Flachseiten des Plättchens 30 sind zum Beispiel aus Kunststoff bestehende Lichtleiter 21a, 21b angeordnet, die mit der Lichtanalyseeinrichtung 13 verbunden sind. An den von dem Plättchen 30 aufragenden Schmalseiten 31 der Lichtleiter 21a, 21b ist der Lichtleiter sichtbar. Die Schmalseiten 31 sind vorzugsweise ebenso wie die Flachseiten des Plättchens 30 hydrophil ausgebildet, so dass sich im Betrieb aus Gewebeflüssigkeit, Spülflüssigkeit oder dergleichen, Fluidkörper 27 bilden, die an dem Lichtleiter 21a, 21b und dem Plättchen 30 durch Adhäsion gehalten sind und die Lichteintrittsfenster 19 bilden. Es können auch Fluidzuführmittel vorgesehen sein, um den Fluidkörper 27 gezielt zu erzeugen oder fortwährend zu ergänzen und zu erneuern.

Das erfindungsgemäße Instrument 11 zur elektrochirurgischen Einwirkung auf biologisches Gewebe umfasst eine Elektrode 18 sowie einen Lichtleiter 21, der mit einem durch einen Fluidkörper 27 gebildeten Lichteintrittsfenster 19 verbunden ist. Der Lichtleiter ist mit einer Lichtanalyseeinrichtung 13 verbunden, um das bei der HF-Chirurgie an der Elektrode 18 entstehende Licht aufzunehmen und der Lichtanalyseeinrichtung 13 zuzuleiten. Das Lichteintrittsfenster 19 ist am Entstehungsort des Lichts, nämlich unmittelbar an der Elektrode, d.h. an dem entstehenden Funken. angeordnet. Eine Verfälschung des aufgenommenen Lichts durch Rauch- oder Partikelniederschlag auf dem Lichteintrittsfenster 19 kann nahezu vermieden werden.

### Bezugszeichenliste:

- 10: elektrochirurgische Einrichtung
- 11: Instrument
- 12: Gerät
- 13: Lichtanalyseeinrichtung
- 14: Leitung
- 15: elektrische Leitung
- 16: Lichtleiter
- 17: Fluidleitung
- 18: Elektrode
- 19: Lichteintrittsfenster
- 20: Elektrodenträger
- 21: starrer Lichtleiter
- 22: Griffs
- 23: Bedienelemente
- 24: Ende des Lichtleiters 21
- 25: Wendelnut
- 26: Draht
- 26a, b: Drahtwindungen, die Bereiche der Elektrode 18 bilden
- 27: Fluidkörper
- 27a: strömender Fluidkörper, austretender Fluidstrahl
- 28: Fluidkanal
- 29: Fluidaustrittsöffnungen
- 30: Plättchen
- 31: Schmalseiten
- 32: Isolierender Werkstoff beispielsweise Kunststoff

## Patentansprüche

1. Instrument (11) zur elektrochirurgischen Einwirkung auf biologisches Gewebe, insbesondere zur HF-Chirurgie
mit einer Elektrode (18), die über eine Leitung (15) mit einer elektrischen Quelle (12) verbindbar ist,
mit einem Lichtleiter (21), der sich von mindestens einem Lichteintrittsfenster (19) ausgehend erstreckt und über den das Instrument (11) an eine Lichtanalyseeinrichtung (13) anschließbar ist,
wobei der Lichtleiter (21) ein Ende (24) aufweist, das die Elektrode (18) trägt.

2. Instrument nach Anspruch 1, **dadurch gekennzeichnet, dass** das Ende (24) eine Kegelspitze ist und dass die Elektrode (18) durch Drahtwindungen (26a, 26b) gebildet ist, zwischen denen die Lichteintrittsfenster (19) ausgebildet sind.

3. Instrument nach einem der vorstehenden Ansprüche, **dadurch gekennzeichnet, dass** das Lichteintrittsfenster (19) durch einen Fluidkörper (27) gebildet ist.

4. Instrument nach einem der vorstehenden Ansprüche, **dadurch gekennzeichnet, dass** das Lichteintrittsfenster (19) in unmittelbarer Nachbarschaft zu der Elektrode (18) angeordnet ist.

5. Instrument nach einem der vorstehenden Ansprüche, **dadurch gekennzeichnet, dass** das Lichteintrittsfenster (19) die Elektrode (18) berührend angeordnet ist.

6. Instrument nach einem der vorstehenden Ansprüche, **dadurch gekennzeichnet, dass** der Fluidkörper (27) durch Adhäsion zwischen der Elektrode (18) und der Flüssigkeit des Fluidkörpers (27a) und/oder durch Adhäsion zwischen der Flüssigkeit des Fluidkörpers (27a) und dem Lichtleiter (21) gehalten ist.

7. Instrument nach einem der vorstehenden Ansprüche, **dadurch gekennzeichnet, dass** das Lichteintrittsfenster (19) und/oder die Elektrode (18) wenigstens abschnittsweise eine hydrophile Oberfläche aufweisen.

8. Instrument nach einem der vorstehenden Ansprüche, **dadurch gekennzeichnet, dass** die Elektrode (18) mindestens zwei elektrisch miteinander verbundene Bereiche (26a, 26b) aufweist, zwischen denen das Lichteintrittsfenster (19) angeordnet ist,

9. Instrument nach einem der vorstehenden Ansprüche, **dadurch gekennzeichnet, dass** das Lichteintrittsfenster (19) sich entlang der Elektrode (18, 26) erstreckend ausgebildet ist.

10. Instrument nach einem der vorstehenden Ansprüche, **dadurch gekennzeichnet, dass** das Lichteintrittsfenster (19) durch einen ruhenden Fluidkörper (27) ausgebildet ist.

11. Instrument nach einem der vorstehenden Ansprüche, **dadurch gekennzeichnet, dass** der Lichtleiter (21) einen starren Abschnitt aufweist.

## Claims

1. Instrument (11) for electrosurgical action on biological tissue, in particular for HF surgery,
with an electrode (18), which can be connected to an electric power source (12) via a line (15),
with a light guide (21), which extends from at least one light entry window (19) and by means of which the instrument (11) can be connected to a light analyser (13), wherein the light guide (21) has an end (24) supporting the electrode (18).

2. Instrument according to claim 1, **characterised in that** the end (24) is a cone point, and that the electrode (18) is formed by wire windings (26a, 26b), between which the light entry window (19) is configured.

3. Instrument according to one of the preceding claims, **characterised in that** the light entry window (19) is formed by a fluid body (27).

4. Instrument according to one of the preceding claims, **characterised in that** the light entry window (19) is arranged in the direct vicinity of the electrode (18).

5. Instrument according to one of the preceding claims, **characterised in that** the light entry window (19) is arranged touching the electrode (18).

6. Instrument according to one of the preceding claims, **characterised in that** the fluid body (27) is held by adhesion between the electrode (18) and the liquid of the fluid body (27a) and/or is held by adhesion between the liquid of the fluid body (27a) and the light guide (21).

7. Instrument according to one of the preceding claims, **characterised in that** the light entry window (19) and/or the electrode (18) have a hydrophilic surface at least in sections.

8. Instrument according to one of the preceding claims, **characterised in that** the electrode (18) has at least two electrically connected regions (26a, 26b), between which the light entry window (19) is arranged.

9. Instrument according to one of the preceding claims, **characterised in that** the light entry window (19) is configured to extend along the electrode (18, 26).

10. Instrument according to one of the preceding claims, **characterised in that** the light entry window (19) is configured by a resting fluid body (27).

11. Instrument according to one of the preceding claims, **characterised in that** light guide (21) has a rigid section.

## Revendications

1. Instrument (11) destiné à exercer une action électrochirurgicale sur un tissu biologique, notamment pour la chirurgie HF,
comprenant une électrode (18) qui peut être reliée par une ligne (15) à une source électrique (12),
comprenant un conducteur de lumière (21) qui s'étend à partir d'au moins une fenêtre d'entrée de lumière (19) et par l'intermédiaire duquel l'instrument (11) peut être connecté à un dispositif d'analyse de lumière (13),
le guide de lumière (21) présentant une extrémité (24) qui porte l'électrode (18).

2. Instrument selon la revendication 1, **caractérisé en ce que** l'extrémité (24) est une pointe de cône et **en ce que** l'électrode (18) est formée de spires de fil métallique (26a, 26b) entre lesquelles sont formées les fenêtres d'entrée de lumière (19).

3. Instrument selon l'une des revendications précédentes, **caractérisé en ce que** la fenêtre d'entrée de lumière (19) est formée par un corps fluide (27).

4. Instrument selon l'une des revendications précédentes, **caractérisé en ce que** la fenêtre d'entrée de lumière (19) est disposée dans le voisinage immédiat de l'électrode (18).

5. Instrument selon l'une des revendications précédentes, **caractérisé en ce que** la fenêtre d'entrée de lumière (19) est disposée de manière à être en contact avec l'électrode (18).

6. Instrument selon l'une des revendications précédentes, **caractérisé en ce que** le corps fluide (27) est maintenu par adhérence entre l'électrode (18) et le liquide du corps fluide (27a) et/ou par adhérence entre le liquide du corps fluide (27a) et le conducteur de lumière (21).

7. Instrument selon l'une des revendications précédentes, **caractérisé en ce que** la fenêtre d'entrée de lumière (19) et/ou l'électrode (18) présentent au moins par portions une surface hydrophile.

8. Instrument selon l'une des revendications précédentes, **caractérisé en ce que** l'électrode (18) présente au moins deux zones (26a, 26b) qui sont connectées électriquement entre elles et entre lesquelles est disposée la fenêtre d'entrée de lumière (19).

9. Instrument selon l'une des revendications précédentes, **caractérisé en ce que** la fenêtre d'entrée de lumière (19) est réalisée de manière à s'étendre le long de l'électrode (18, 26).

10. Instrument selon l'une des revendications précédentes, **caractérisé en ce que** la fenêtre d'entrée de lumière (19) est formée par un corps fluide (27) au repos.

11. Instrument selon l'une des revendications précédentes, **caractérisé en ce que** le conducteur de lumière (21) présente un tronçon rigide.
